# EUROPEAN PATENT SPECIFICATION

(11) **EP 3 574 900 B1**
(45) Date of publication and mention of the grant of the patent: **07.04.2021**
(21) Application number: 18760546.4
(22) Date of filing: 22.02.2018
(51) Int. Cl.: A61K 31/438, A61K 9/70, A61K 9/48, A61K 9/20, A61K 9/02, A61K 9/00, A61P 31/04, A61P 15/02, A61K 47/54, A61K 9/06, A61K 47/10, A61K 47/32

(54) **NEW APPLICATIONS OF RIFAMYCIN-NITROIMIDAZOLE COUPLING MOLECULES**
NEUE ANWENDUNGEN VON RIFAMYCIN-NITROIMIDAZOL-KUPPLUNGSMOLEKÜLEN
NOUVELLE APPLICATION D'UNE MOLÉCULE DE COUPLAGE DE RIFAMYCINE-NITROIMIDAZOLE

(30) Priority: 28.02.2017 CN 201710109978
(43) Date of publication of application: 04.12.2019
(73) Proprietor: Tennor Therapeutics Limited, Suzhou, Jiangsu 215123 (CN)
(72) Inventor: MA, Zhenkun, Suzhou Jiangsu 215123 (CN); YUAN, Ying, Suzhou Jiangsu 215123 (CN); LIU, Yu, Suzhou Jiangsu 215123 (CN); WANG, Xiaomei, Suzhou Jiangsu 215123 (CN)
(74) Representative: Gong, Jinping
(86) International application number: PCT/CN2018/076969
(87) International publication number: WO 2018/157750

(56) References cited:
- WO-A2-2008/008480
- CN-A- 104 971 061
- CN-A- 105 037 389
- CN-A- 106 860 451
- US-A1- 2016 038 468
- DATABASE MEDLINE [Online] US NATIONAL LIBRARY OF MEDICINE (NLM), BETHESDA, MD, US; 1983, BALSDON M J: "Treatment of the Gardnerella vaginalis syndrome with a single 2 gram oral dosage of metronidazole.", XP002796504, Database accession no. NLM6607515 & SCANDINAVIAN JOURNAL OF INFECTIOUS DISEASES. SUPPLEMENTUM 1983, vol. 40, 1983, pages 101-102, ISSN: 0300-8878
- Ayesha B M Kharsany ET AL: "for Microbiology NOTES Antimicrobial Susceptibilities of Gardnerella vaginalis", ANTIMICROBIAL AGENTS AND CHEMOTHERAPY, 1 December 1993 (1993-12-01), pages 2733-2735, XP055652927, Retrieved from the Internet: URL:https://aac.asm.org/content/aac/37/12/ 2733.full.pdf

## Description

### Technical Field

The present invention belongs to the field of medical chemistry, and particularly relates to a new use of a rifamycin-nitroimidazole conjugate molecule.

### Background

Anaerobic bacteria belong to a type of bacteria that can grow better in an anaerobic condition than in an aerobic environment but cannot grow on the surface of solid culture media at the concentration of air (18% oxygen) and/or 10% carbon dioxide. This type of bacteria lack a complete metabolic enzyme system, so that the energy metabolism thereof is performed in an anaerobic fermentation manner.

Anaerobic bacteria include the following types:

### 1. Gram-positive anaerobic cocci (GPAC)

Include *Peptostreptococcus, Finegoldia, Anaerococcus, Peptoniphilus, Veillonella* and the like, constitute a part of the microbial flora of human oral cavity, upper respiratory tract, gastrointestinal tract and female genitourinary system, are opportunistic pathogens, may cause different degrees of infections in various parts of the human body (accounting for 25-30% of all anaerobic bacteria infections), including mild skin suppuration or severe brain and epidural abscess, bacteremia, endocarditis, necrotizing pneumonia and septic abortion. GPAC may cause up to 40% of pleural/pulmonary infections, and the mortality rate may be up to 75% among the severe wound infections caused thereby. GAPC may also cause eye, facial features, head and neck infections, meningitis, pericarditis, femoral and joint (including artificial joint) infections, breast abscess, urinary tract infection, etc.

### 2. Gram-negative anaerobic cocci (GNPC)

GNPC is one of the normal bacteria of human oral cavity, genitourinary tract, respiratory tract and intestinal tract, the proportion thereof in clinical samples is very low, but has an increasing trend.

### 3. Non-spore-forming anaerobic Gram-positive rods

Include *Propionibacterium, Lactobacillus, Actinomyces, Eubacterium, Eggerthella, Atopobium, Bifidobacterium* and *Mobiluncus;* mainly cause compound infections related to mucosae, wherein oral cavity and feces are the main infection sources; and is the major cause of postoperative mortality and morbidity.

### 4. Anaerobic Gram-negative rods

Include *Bacteroides, Porphyromonas, Prevotella* and *Fusobacterium,* and are mainly parasitic on human oral cavity, gastrointestinal tract and vagina mucosae, wherein infections are caused by mucosa damage in general, for example vaginitis and periodontitis.

### 5. Spore-forming Gram-positive rods

Include *Clostridium,* and may cause clostridium bacteremia, food poisoning, necrotic enteritis, Iatrogenic diarrhea (CDI), pseudomembranous colitis associated with antibiotics, skin and soft tissue infections.

As a common infectious disease of lower genital tract in women of childbearing age, *Bacterial Vaginosis* (BV) is a syndrome without vaginal mucosal inflammation caused by changes in composition of the normal vaginal microecological flora. Millions of women worldwide suffer from bacterial vaginosis every year, seriously affecting health. BV may cause adverse pregnancy outcomes such as spontaneous abortion, premature delivery, amniotic fluid infection, puerperal endometritis, caesarean section wound infection and perinatal complication. In addition, the recurrence and persistent infection of BV may also increase the risk of trichomonas vaginitis, vulvovaginal candidiasis, cervical cancer and human immunodeficiency virus (HIV) infection.

*Gardnerella vaginalis* (GV) is one of the main causes of BV, Metronidazole is still the firstline drug for clinical conventional treatment of BV, although the short-term cure rate for BV may reach 70% to 80%, the recurrence rate within 3 months may be up to 58%. The presence of the metronidazole drug-resistant strain of GV and the formation of biofilm may be important causes of BV recurrence and treatment failure. Studies have shown that the response of bacteria in the biofilm to antibacterial drugs is significantly different from the planktonic growth pattern thereof, which may be related to the special growth state of the bacteria in the biofilm and the penetrating power of the antibacterial drugs reduced by the biofilm. Therefore, the discovery of the structure of biofilm produced by *Gardnerella* is a new hot topic in the research of BV recurrence and drug resistance.

The U.S. Patent No.7,678,791 B2 discloses a compound 4-deoxy-3,4-[2-spiro-[2-(2-methyl-5-nitro-imidazole-1-yl) ethyl]-piperidine-4-yl]-(1-hydrogen)-imidazo-(2,5-dihydro) rifamycin S which has antimicrobial activity against several bacteria such as *Escherichia coli,* etc., but has no documented antibacterial activity against the anaerobic bacteria *Gardnerella vaginalis.*

US 2016/038468 A1 describes rifaximin for use in the treatment of bacterial vaginal infections, including those characterised by the presence of bacteria that may be clindamycin and/or metronidazole resistant. Rifaximin can thus be combined with clindamycin or metronidazole for use in the treatment of a bacterial infection.

### Summary

In view of the above defects existing in the prior art, the object of the present invention is to provide a new use of a rifamycin-nitroimidazole conjugate molecule which may be effectively against anaerobic bacteria, and may be used to treat bacterial vaginosis.

The object of the present invention is realized by the following technical solution:
A rifamycin-nitroimidazole conjugate molecule shown in formula I for use in treating bacterial vaginosis caused by Gardnerella vaginalis as specified in any of claims 1 to 3. for use in preparing a pharmaceutical composition for use in treating bacterial vaginosis caused by Gardnerella vaginalis as specified in any of claims 1 to 3.

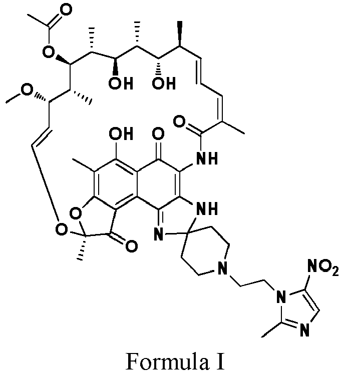

Preferably, in the use, the anaerobic bacteria flora comprises one or a combination of *Actinomyces naeslundii, Anaerococcus prevotii, Atopobium vaginae, Bacteroides fragilis* (including MET^{R}), *Bacteroides thetaiotaomicron* (including MET^{R}), *Bacteroides gracilis, Bacteroides uniformis, Bacteroides vulgatus, Bacteroides ovatus* (including MET^{R}), *Bifidobacterium breve, Bifidobacterium longum, Clostridium sporogenes, Clostridium perfringens* (including MET^{R}), *Eubacterium rectale, Fusobacterium nucleatum, Gardnerella vaginalis, Lactobacillus crispatus, Lactobacillus gasseri, Lactobacillus jensenii, Mobiluncus* (curtisii subsp. curtisii), *Mobiluncus mulieris, Peptococcus, Peptoniphilus asaccharolyticus* (including MET^{R}), *Peptostreptococcus, Peptostreptococcus anaerobius, Prevotella bivia* (including MET^{R}), *Prevotella disiens, Prevotella intermedia, Treponema denticola* and *Veionella parvula.*

The present invention further provides a pharmaceutical composition comprising the rifamycin-nitroimidazole conjugate molecule for use in treating Bacterial Vaginosis (BV) caused by Gardnerella vaginalis flora imbalance as specified in any of claims 4 to 6.

Preferably, in the use, the human effective dose of the rifamycin-nitroimidazole conjugate molecule is 10mg-10g per day, and the treatment period is 1-15 days.

Preferably, in the use, the administration route used includes one or a combination of injection administration, oral administration, intracavitary administration, enteral administration, and transdermal absorption.

Preferably, in the use, the administration dosage form used includes one or a combination of injection, suppository, tablet, capsule, patch and extended release dosage form.

The present invention has the prominent effects: the rifamycin-nitroimidazole conjugate molecule shown in formula I of the present invention has broad-spectrum antibacterial activity, including activity against most vaginal pathogenic bacteria, has in vitro antibacterial activity stronger than that of drugs against BV such as metronidazole and clindamycin, and has potential use in prevention and treatment of bacterial vaginosis caused by identified anaerobic bacteria genera and species and the above other anaerobic bacteria infections.

In order to make the technical solution of the present invention easier to understand and master, the specific embodiments of the present invention will be described in further detail below with reference to the examples.

### Detailed Description

The present invention is further described below by way of specific embodiments. However, the present invention is not limited to the specific embodiments. The experimental methods described in the following embodiments are conventional methods unless otherwise specified; and the reagents and materials are commercially available unless otherwise specified.

### Embodiment 1

This embodiment provides a use of a rifamycin-nitroimidazole conjugate molecule shown in formula I against anaerobic bacteria; 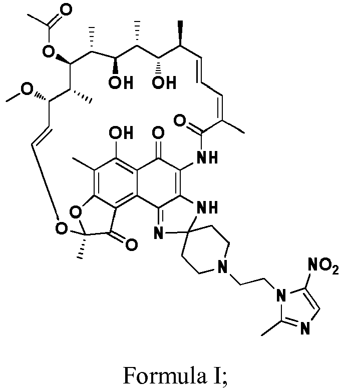 wherein the anaerobic bacteria comprise one or a combination of *Actinomyces naeslundii, Anaerococcus prevotii, Atopobium vaginae, Bacteroides fragilis* (including MET^{R}), *Bacteroides thetaiotaomicron* (including MET^{R}), *Bacteroides gracilis, Bacteroides uniformis, Bacteroides vulgatus, Bacteroides ovatus* (including MET^{R}), *Bifidobacterium breve, Bifidobacterium longum, Clostridium sporogenes, Clostridium perfringens* (including MET^{R}), *Eubacterium rectale, Fusobacterium nucleatum, Gardnerella vaginalis, Lactobacillus crispatus, Lactobacillus gasseri, Lactobacillus jensenii, Mobiluncus* (curtisii subsp. curtisii), *Mobiluncus mulieris, Peptococcus (niger), Peptoniphilus asaccharolyticus* (including MET^{R}), *Peptostreptococcus, Peptostreptococcus anaerobius, Prevotella bivia* (including MET^{R}), *Prevotella disiens, Prevotella intermedia, Treponema denticola* and V*eionella parvula.*

In this embodiment, all the tests of the rifamycin-nitroimidazole conjugate molecule shown in formula I on pathogenic bacteria associated with bacterial vaginosis are performed using the agar dilution method consistent with that in the Guideline of the Clinical and Laboratory Standards Institute (CLSI; 1-3). All the tests are performed under anaerobic conditions. Control compounds include metronidazole, rifampicin and clindamycin.

### Material and Method

### Test Compounds

Provided by TenNor Therapeutics Ltd., and stored at -20°C before test. Three control drugs are provided by Sigma. All stock liquors are allowed to stand for at least 1 hour before being automatically sterilized.

### Test Strains

The tested clinical isolates may be reference strains obtained from American Type Culture Collection, ATCC, Manassas, VA. After being received, the strains are respectively inoculated on appropriate agar plates and placed under optimized conditions for growth. The growing strains are cloned in the broth containing cryoprotectant to prepare bacterial suspensions, and the bacterial suspensions are subpackaged and then stored in freezing at -80°C. Before test, the frozen strains are inoculated into appropriate agar dishes and cultured for growth. Anaerobic bacteria grow for 48 hours at 35°C in a Bactron II oxygen-free cabinet (Shel Lab, Cornelius, OR) before test.

### Test Broths

The broth used for drug sensitivity detection by the anaerobic agar dilution method is supplementary Brucella agar (SBA) composed of Brucella agar containing 5 µg/mL of sanguine (BD/BBL; Art. No.: 5300551), 1 µg/mL of vitamin k1 (Sigma, St. Louis, MO; Art. No.: SLBC4685V) and 5% lake sheep blood (Cleveland Scientific, Bath, OH; Art. No.: 291958).

Preparation and storage of all the above broths are performed in accordance with CLSI (1-3).

The Minimum Inhibitory Concentration (MIC) is determined using the agar dilution method.

The MIC values of all microorganisms except haemophilus are determined using the agar dilution method in the CLSI (1-2). Drugs are manually diluted and agar plates containing drugs are prepared in accordance with the CLSI guideline (1-2). To dry the agar surface, a multi-well plate is plated at room temperature for 1 hour. The agar plate used for testing under anaerobic condition is pre-placed in an oxygen-free cabinet for about 1 hour. All isolates are adjusted to 0.5 McFarland Standard in appropriate broths using a nephelometer (Dade Behring MicroScan, Wet Sacramento, CA). Then, each bacterial suspension is transferred into wells of the test plate using a stainless steel duplicator. About 10⁵/1-2 microliters of bacteria are inoculated on the agar surface in each well, and after drying, the drug plate and the drug-free control plate are placed in the oxygen-free cabinet to be cultured at 35°C for 42-48 hours. The MIC is determined in accordance with the CLSI guideline (1-2).

The test results are shown in Table 1.

**Table 1**

| **Strain Name** | **No.** | **MIC (µg/mL)** | | | |
|---|---|---|---|---|---|
| | | **Compound I** | **Metronidazole** | **Clindamycin** | **Rifampicin** |
| *Actinomyces naeslundii* | ATCC 12104 | 0.001 | 256 | 0.5 | 0.03 |
| *Anaerococcus (Anaerococcus(prevotii)*) | ATCC 9321 | 0.0005 | 2 | 0.06 | 0.03 |
| *Atopobium vaginae* | BAA-55 | 0.03 | 128 | 0.008 | 0.25 |
| *Bacteroides fragilis* (QC) | ATCC 25285 | 0.03 | 1 (0.25-1)* | 1 (0.25-2) | 0.25 |
| *Bacteroides fragilis* (MET^{R}) | MMX 3387 | 0.015 | >256 | 2 | 0.25 |
| *Bacteroides thetaiotaomicron* (MET^{R}) | MMX 3409 | 0.03 | 2 | >64 | 0.5 |
| *Bacteroides gracilis* | ATCC 33236 | 0.5 | >256 | 0.06 | 16 |
| *Bacteroides uniformis* | MMX 1277 | 0.03 | >256 | 0.25 | 0.5 |
| *Bacteroides vulgatus* | MMX 8348 | 0.03 | 1 | 0.25 | 0.25 |
| *Bacteroides vulgatus* (MET^{R)} | MMX 3490 | 0.03 | 128 | 64 | 0.25 |
| *Bacteroides ovatus* (MET^{R}) | MMX 3504 | 0.12 | 2 | 8 | 1 |
| *Bifidobacterium (breve)* | ATCC 15698 | 0.015 | 8 | 0.03 | 0.25 |
| *Bifidobacterium longum* | ATCC 15707 | 0.015 | 8 | 0.008 | 0.5 |
| *Clostridium sporogenes* | ATCC 19404 | 0.015 | 0.06 | 8 | 1 |
| *Clostridium perfringens (MET^{R})* | MMX 3521 | 2 | >256 | 64 | 1 |
| *Eubacterium (rectale)* | ATCC 33656 | 0.0005 | 0.5 | 0.008 | 0.015 |
| *Fusobacterium nucleatum* | ATCC 10953 | 0.12 | 0.06 | 0.03 | 1 |
| *Fusobacterium nucleatum* | ATCC 25586 | 0.001 | 2 | 0.03 | 0.5 |
| *Gardnerella vaginalis* | ATCC 14018 | 0.004 | 4 | 0.06 | 0.5 |
| *Gardnerella vaginalis* | ATCC 49145 | 0.004 | 4 | 0.06 | 0.5 |
| *Lactobacillus crispatus* | ATCC 33820 | 4 | >256 | 64 | 2 |
| *Lactobacillus gasseri* | ATCC 33323 | 0.015 | >256 | 4 | 0.25 |
| *Lactobacillus jensenii* | ATCC 25258 | 0.008 | >256 | 0.5 | 0.5 |
| *Mobiluncus (curtisii subsp. curtisii)* | ATCC 35241 | 0.002 | 2 | 0.06 | 0.004 |
| *Mobiluncus (mulieris)* | ATCC 35243 | 0.001 | 0.5 | 0.03 | 0.004 |
| *Peptococcus (niger)* | ATCC 27731 | 0.0005 | 0.5 | 0.03 | 0.004 |
| *Peptoniphilus asaccharolyticus* | ATCC 29743 | 0.008 | 0.5 | 4 | 0.004 |
| *Peptostreptococcus (magnus)* | ATCC 14956 | 0.0005 | 1 | 1 | 1 |
| *Peptostreptococcus anaerobius* | ATCC 27337 | 0.002 | 0.25 | 0.12 | 0.004 |
| *Prevotella asaccharolytica (MEJ^{R})* | MMX 3552 | 0.008 | 1 | 32 | 0.004 |
| *Prevotella bivia* | ATCC 29303 | 0.015 | 1 | 0.03 | 0.5 |
| *Prevotella bivia* | MMX 3450 | 0.0005 | 1 | 16 | 0.06 |
| *Prevotella bivia* (MET^{R}) | MMX 3454 | 0.015 | 0.5 | 0.06 | 0.004 |
| *Prevotella disiens* | MMX 3457 | 0.008 | 0.5 | 0.25 | 0.5 |
| *Prevotella disiens* | MMX 3446 | 0.015 | 0.5 | 0.12 | 0.12 |
| *Prevotella intermedia* | ATCC 25611 | 0.0005 | 1 | 0.008 | 0.12 |
| *Treponema denticola* | ATCC 35405 | 0.002 | 0.5 | 0.12 | 0.004 |
| *Veionella parvula* | ATCC 17745 | 2 | 2 | 32 | 4 |

As shown in the above test results, except having activity against a few strains which is equivalent to the anti-anaerobe drug-metronidazole most commonly used at present, the rifamycin-nitroimidazole conjugate molecule (formula I) has in vitro antibacterial activity (MIC) against most anaerobic bacteria which is about 100 to 1000 times higher than that of metronidazole. It has extremely strong antibacterial activity (MIC=0.03-0.0005 ug/mL) against common BV dominant bacteria such as *Gardnerella vaginalis, Prevotella, Mobiluncus, Atopobium vaginae* and *Peptostreptococcus.* The rifamycin-nitroimidazole conjugate molecule (formula I) has significantly stronger antibacterial effect (MIC=0.004 micrograms/ml) on Gardnerella as major BV bacteria than the two parent antibiotics, i.e. metronidazole (MIC=4 ug/mL) and rifampicin (MIC= 0.5 ug/mL), showing a strong synergistic effect between two covalently coupled functional groups in the structure of compound I. From the broad-spectrum antibacterial activity of the rifamycin-nitroimidazole conjugate molecule (formula I), it is predicted that the rifamycin-nitroimidazole conjugate molecule may also have a good efficacy on infections caused by other bacteria tested here.

According to the MIC value of the rifamycin-nitroimidazole conjugate molecule (formula I), it is predicted that the effective dose of the rifamycin-nitroimidazole conjugate molecule for bacterial vaginosis is 1/100 of that of metronidazole, which is equivalent to 10mg per day. In order to achieve a better drug effect, the dose of the rifamycin-nitroimidazole conjugate molecule (formula I) may be continuously increased to 10g to reach the highest effective dose thereof.

### Embodiment 2

This embodiment provides a formula and preparation method for an immediate release oral dosage form of the rifamycin-nitroimidazole conjugate molecule shown in formula I.

Rifamycin-nitroimidazole conjugate molecule shown in formula I 100g

| | |
|---|---|
| Mannitol | 154g |
| Sodium starch glycolate | 20g |
| Polyvinyl pyrrolidone K30 | 9g |
| Sodium dodecyl sulfate | 3g |
| Silicon dioxide | 8g |
| Magnesium stearate | 6g |
| Purified water | Appropriate amount |
| Prepared in total | 1000 EA |

Weighing the rifamycin-nitroimidazole conjugate molecule shown in formula I and excipients according to the formula; dissolving Polyvinyl Pyrrolidone K30 (PVP K30) and sodium dodecyl sulfate (SDS) in purified water, stirring for 1 hour, and taking the stirred product as binder for later use; sieving the rifamycin-nitroimidazole conjugate molecule shown in formula I, mannitol and sodium starch glycolate (DST) with a sieve of 30 meshes, adding the mixture into a granulator for premixing, wherein the impeller stirring speed is 700rpm, and the time duration is about 15 minutes; using a peristaltic pump to add an appropriate amount of purified water and adhesive into the granulator mixture at a fixed speed (145-165 g/ min), wherein the stirring speed of the granulator impeller is 400 rpm, and the time duration is about 1-2 minutes, and continuing to mix for 0.5-1 minute after the adhesive is completely added; drying the wet particles using a fluid bed, supposing that the air inlet temperature is 60°C, and the air inlet rate is 40m³/h; according to the weight of the dried dry particle material, calculating the weight of silicon dioxide and magnesium stearate to be added, placing the silicon dioxide and dry particles in a bin blender for mixing, wherein the mixing time duration 15 minutes, and the speed is 20 rpm; then adding magnesium stearate, wherein the mixing time duration is 6 minutes at 20 rpm, taking the totally mixed material to fill No. 0 capsules using a capsule filling machine, and then obtaining hard capsules of the rifamycin-nitroimidazole conjugate molecule shown in formula I.

Tableting the totally mixed material using a tableting machine, and then obtaining tablets of the rifamycin-nitroimidazole conjugate molecule shown in formula I.

### Embodiment 3

This embodiment provides a preparation method for injections of the rifamycin-nitroimidazole conjugate molecule shown in formula I.

Rifamycin-nitroimidazole conjugate molecule shown in formula I 30g

| | |
|---|---|
| Mannitol | 20g |
| Sodium formaldehyde sulfoxylate | 0.5g |
| Tween-80 | 0.1g |
| IN NaOH | 36 mL |
| Water for injection | Added to 1000mL |

Adding mannitol, sodium formaldehyde sulfoxylate and Tween-80 into an appropriate amount of water for injection under the protection of nitrogen, adding the rifamycin-nitroimidazole conjugate molecule shown in formula I, stirring for 10-15 minutes at the intermediate speed, wetting the rifamycin-nitroimidazole conjugate molecule shown in formula I, slowing adding IN NaOH dropwise, wherein about 175 minutes are consumed (rapid at first and slow down then), until the rifamycin-nitroimidazole conjugate molecule shown in formula I is completely dissolved; filtering using two microporous membranes of 0.45+0.22µm, filling the filtrate into 10mL glass bottles, each bottle being filled with 3.5mL, transferring the glass bottles into a freeze dryer for freeze-drying, and obtaining freeze-dried powder for injections of the rifamycin-nitroimidazole conjugate molecule shown in formula I after screwing caps.

### Embodiment 4

This embodiment provides a preparation method for gels for external use of the rifamycin-nitroimidazole conjugate molecule shown in formula I.

Rifamycin-nitroimidazole conjugate molecule shown in formula I 2g

| | |
|---|---|
| Carbomer | 5g |
| Natural borneol | 1.6g |
| Surfactant | 1.2g |
| Ethanol | 200mL |
| Purified water | Added to 2000mL |

Wetting carbomer with an appropriate amount of ethanol, diluting to about 1600mL with purified water under the stirring condition, boiling, continuously stirring to form transparent gel solution, and cooling; taking the rifamycin-nitroimidazole conjugate molecule shown in formula I, natural borneol and essence, dissolving with ethanol and adding into the transparent gel solution together with surfactant, filling with purified water to the volume of 2000mL, stirring uniformly, standing still for more than 12 hours, defoaming at normal pressure, subpackaging after inspection, and obtaining gels for external use of the rifamycin-nitroimidazole conjugate molecule shown in formula I.

## Claims

1. A rifamycin-nitroimidazole conjugate molecule shown in formula I for use in treating bacterial vaginosis caused by anaerobic bacteria, 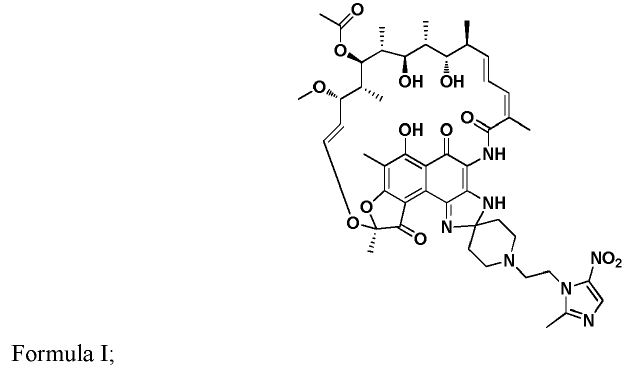 and
**characterized in that** the human effective dose of the rifamycin-nitroimidazole conjugate molecule is 10 mg-10g per day, and the treatment period is 1-15 days; and wherein the anaerobic bacteria is Gardnerella vaginalis.

2. The rifamycin-nitroimidazole conjugate molecule for use according to claim 1, **characterized in that** the administration route used includes one or a combination of injection administration, oral administration, intracavitary administration, enteral administration, and transdermal absorption.

3. The rifamycin-nitroimidazole conjugate molecule for use according to claim 1, **characterized in that** the administration dosage form used includes one or a combination of injection, suppository, tablet, capsule, patch and extended release dosage form.

4. A pharmaceutical composition comprising the rifamycin-nitroimidazole conjugate molecule according to claim 1 for use in treating bacterial vaginosis caused by anaerobic bacteria;
**characterized in that** the human effective dose of the rifamycin-nitroimidazole conjugate molecule is 10 mg-10g per day, and the treatment period is 1-15 days.

5. The pharmaceutical composition comprising rifamycin-nitroimidazole conjugate molecule for use according to claim 4, **characterized in that** the administration route used includes one or a combination of injection administration, oral administration, intracavitary administration, enteral administration, and transdermal absorption.

6. The pharmaceutical composition comprising rifamycin-nitroimidazole conjugate molecule for use according to claim 4, **characterized in that** the administration dosage form used includes one or a combination of injection, suppository, tablet, capsule, patch and extended release dosage form.

## Patentansprüche

1. Ein in Formel I gezeigtes Rifamycin-Nitroimidazol-Konjugatmolekül zur Behandlung der durch ein anaerobes Bakterium verursachten bakteriellen Vaginose, 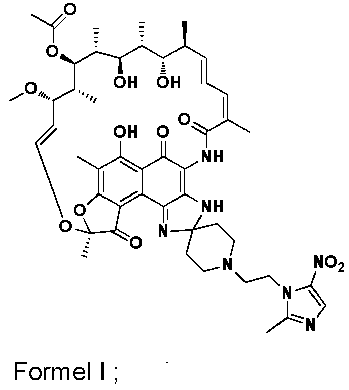 und
**dadurch gekennzeichnet, dass** die vom Menschen wirksame Dosis des Rifamycin-Nitroimidazol-Konjugatmoleküls 10 mg bis 10 g pro Tag beträgt und die Behandlungsdauer 1 bis 15 Tage beträgt; und wobei das anaerobe Bakterium Gardnerella vaginalis ist.

2. Das Rifamycin-Nitroimidazol-Konjugatmolekül zur Verwendung nach Anspruch 1, **dadurch gekennzeichnet, dass** die verwendete Verabreichungsweg eine oder eine Kombination aus Injektionsverabreichung, oraler Verabreichung, intrakavitärer Verabreichung, enteraler Verabreichung und transdermaler Absorption umfasst.

3. Das Rifamycin-Nitroimidazol-Konjugatmolekül zur Verwendung nach Anspruch 1, **dadurch gekennzeichnet, dass** die verwendete Verabreichungsdosierungsform eine oder eine Kombination aus Injektions-, Zäpfchen-, Tabletten-, Kapsel-, Pflaster- und verlängerter Freisetzung-Dosierungsform umfasst.

4. Pharmazeutische Zusammensetzung, umfassend das Rifamycin-Nitroimidazol-Konjugatmolekül nach Anspruch 1 zur Behandlung der durch ein anaerobes Bakterium verursachten bakteriellen Vaginose;
**dadurch gekennzeichnet, dass** die vom Menschen wirksame Dosis des Rifamycin-Nitroimidazol-Konjugatmoleküls 10 mg bis 10 g pro Tag beträgt und die Behandlungsdauer 1 bis 15 Tage beträgt.

5. Die pharmazeutische Zusammensetzung, umfassend das Rifamycin-Nitroimidazol-Konjugatmolekül zur Verwendung nach Anspruch 4, **dadurch gekennzeichnet, dass** der verwendete Verabreichungsweg eine oder eine Kombination aus Injektionsverabreichung, oraler Verabreichung, intrakavitärer Verabreichung, enteraler Verabreichung und transdermaler Absorption umfasst.

6. Die pharmazeutische Zusammensetzung, umfassend das Rifamycin-Nitroimidazol-Konjugatmolekül zur Verwendung nach Anspruch 4, **dadurch gekennzeichnet, dass** die verwendete Verabreichungsdosierungsform eine oder eine Kombination aus Injektions-, Zäpfchen-, Tabletten-, Kapsel-, Pflaster- und verlängerter Freisetzung-Dosierungsform umfasst.

## Revendications

1. Molécule conjuguée de rifamycine-nitroimidazole représentée dans la formule I à utiliser dans le traitement de la vaginose bactérienne causée par des bactéries anaérobies, 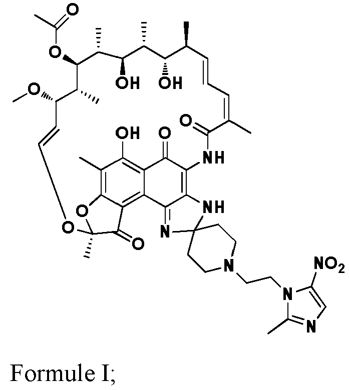 et
**caractérisé en ce que** la dose efficace humaine de la molécule conjuguée de rifamycine-nitroimidazole est de 10 mg à 10 g par jour, et la période de traitement est de 1 à 15 jours, et dans lequel la bactérie anaérobie est Gardnerella vaginalis.

2. Molécule conjuguée rifamycine-nitroimidazole à utiliser selon la revendication 1, **caractérisée en ce que** la voie d'administration utilisée comprend une ou une combinaison d'administration par injection, d'administration orale, d'administration intracavitaire, d'administration entérale et d'absorption transdermique.

3. Molécule conjuguée rifamycine-nitroimidazole à utiliser selon la revendication 1, **caractérisée en ce que** la forme galénique d'administration utilisée comprend une ou une combinaison d'injection, de suppositoire, de comprimé, de capsule, de patch et de forme galénique à libération prolongée.

4. Composition pharmaceutique comprenant la molécule conjuguée de rifamycine-nitroimidazole selon la revendication 1, à utiliser dans le traitement de la vaginose bactérienne causée par des bactéries anaérobies;
**caractérisé en ce que** la dose efficace humaine de la molécule conjuguée de rifamycine-nitroimidazole est de 10 mg à 10 g par jour et la période de traitement est de 1 à 15 jours.

5. Composition pharmaceutique comprenant une molécule conjuguée de rifamycine-nitroimidazole à utiliser selon la revendication 4, **caractérisée en ce que** la voie d'administration utilisée comprend une ou une combinaison d'administration par injection, d'administration orale, d'administration intracavitaire, d'administration entérale et d'absorption transdermique.

6. Composition pharmaceutique comprenant une molécule conjuguée de rifamycine-nitroimidazole à utiliser selon la revendication 4, **caractérisée en ce que** la forme galénique d'administration utilisée comprend une ou une combinaison d'injection, de suppositoire, de comprimé, de capsule, de patch et de forme galénique à libération prolongée.
